# EUROPÄISCHE PATENTSCHRIFT

(11) **EP 3 099 330 B1**
(45) Veröffentlichungstag und Bekanntmachung des Hinweises auf die Patenterteilung: **14.03.2018**
(21) Anmeldenummer: 15709819.5
(22) Anmeldetag: 29.01.2015
(51) Int. Cl.: A61K 47/12, A61K 31/5575

(54) **STABILE ALKOHOLISCHE LÖSUNG VON ALPROSTADIL**
STABLE ALCOHOLIC SOLUTION OF ALPROSTADIL
SOLUTION ALCOHOLIQUE STABLE D'ALPROSTADIL

(30) Priorität: 30.01.2014 AT 500632014
(43) Veröffentlichungstag der Anmeldung: 07.12.2016
(60) Teilanmeldung: 18150928.2
(73) Patentinhaber: Gebro Holding GmbH, 6391 Fieberbrunn (AT)
(72) Erfinder: HÄUSLER, Franz, 83435 Bad Reichenhall (DE); SCHEIDL, Helmut, A-6391 Fieberbrunn (AT); WAGNER, Daniel, A-6380 St. Johann in Tirol (AT)
(74) Vertreter: Wildhack & Jellinek
(86) Internationale Anmeldenummer: PCT/AT2015/050029
(87) Internationale Veröffentlichungsnummer: WO 2015/113094

(56) Entgegenhaltungen:
- CN-A- 103 110 579
- "Pridax Konzentrat zur Herstellung einer Infusionslösung", , 1. August 2013 (2013-08-01), XP055196343, Gefunden im Internet: URL:http://www.pharmore.de/pdf_files/pharm ore-plus/pridax_fi_aug_2013.pdf [gefunden am 2015-06-17]
- YOUNGER E W ET AL: "The stability of prostaglandin E1 in dilute physiological solutions at 37 degrees C", PROSTAGLANDINS, BUTTERWORTH, STONEHAM, MA, US, Bd. 31, Nr. 5, 1. Mai 1986 (1986-05-01), Seiten 923-927, XP025423268, ISSN: 0090-6980, DOI: 10.1016/0090-6980(86)90024-9 [gefunden am 1986-05-01]
- R A UEBEL ET AL: "Stability evaluation of a prostaglandin E1 saline solution packed in insulin syringes", INTERNATIONAL JOURNAL OF IMPOTENCE RESEARCH, Bd. 13, Nr. 1, 1. Februar 2001 (2001-02-01), Seiten 16-17, XP055196348, ISSN: 0955-9930, DOI: 10.1038/sj.ijir.3900625

## Beschreibung

Alprostadil (PGE1) ist ein im Europäischen Arzneibuch beschriebener Wirkstoff aus der Gruppe der Prostaglandin Analoga. Es ist identisch mit dem körpereigenen Prostaglandin E1. Seine hauptsächlichen physiologischen Effekte bestehen in einer Vasodilatation und in einer Hemmung der Thrombozytenaggregation. Aus diesem Wirkprofil leiten sich als die wichtigsten Indikationen die Behandlung erektiler Dysfunktion, die Behandlung peripherer arterieller Verschlusskrankheiten und die kurzzeitige Behandlung bestimmter Herzfehler beim Neugeborenen ab. Als pharmazeutische Darreichungsformen stehen dafür hauptsächlich parenterale, zur Behandlung der erektilen Dysfunktion auch topische Arzneiformen zur Verfügung.

Alprostadil ist ein chemisch außerordentlich instabiler Wirkstoff. Als wichtigste Abbaureaktion nennt der Kommentar des Europäischen Arzneibuchs die Abspaltung von Wasser zu Prostaglandin A₁ (PGA1). Dieses Abbauprodukt wird in der Monographie des Europäischen Arzneibuchs als Verunreinigung A bezeichnet. Sie kann in einer Isomerisierungsreaktion weiter zu Prostaglandin B₁ reagieren, das in der Monographie des Europäischen Arzneibuchs als Verunreinigung B bezeichnet wird. Insgesamt nennt das Europäische Arzneibuch elf Verunreinigungen mit ihrer jeweiligen Molekülstruktur. Gemäß dem Kommentar des Europäischen Arzneibuchs findet die Startreaktion des Abbaus, nämlich die Wasserabspaltung zu PGA1, besonders leicht unter sauren oder basischen Bedingungen statt, wobei in ethanolischer Lösung zwei- und dreiwertige Kationen die Stabilität zusätzlich verschlechtern.

Wegen der Instabilität des Wirkstoffes ist es nicht möglich, gebrauchsfertige parenterale Zubereitungen herzustellen, die eine für die Vermarktung ausreichende Lagerstabilität aufweisen. Alprostadil zur parenteralen Anwendung wird deshalb ausschließlich in Form von Konzentraten angeboten, die kurz vor der Anwendung durch Auflösung in einer geeigneten sterilen Lösung (z.B. physiologische Kochsalzlösung) zur gebrauchsfertigen Infusionslösung verdünnt bzw. rekonstituiert werden. Als Konzentrate werden im Wesentlichen entweder Lyophilisate oder nicht wässrige Lösungen verwendet.

Lyophilisate sind feste, pulverförmige Konzentrate, die durch Gefriertrocknung des Wirkstoffes zusammen mit stabilisierenden Hilfsstoffen hergestellt werden. Der Herstellprozess ist aufwändig und teuer; das Lyophilisat wird entweder in Vials oder anwenderfreundlicher in Zweikammerfertigspritzen oder Doppelkammerkarpulen verpackt und kann bei Raumtemperatur gelagert werden. Als stabilisierender Hilfsstoff wird hauptsächlich alpha-Cyclodextrin (Alfadex) verwendet. Handelsbezeichnungen solcher Produkte sind z.B. Caverject® (Pfizer, Pharmacia), Prostavasin® (UCB Pharma) oder Viridal® (UCB Pharma). Die CN 102688203 beschreibt ein Lyophilisat enthaltend Alprostadil bei dem zur Stabilisierung eine Mischung von Dextran und Hydroxypropyl-beta-Cyclodextrin verwendet wird.

Als nicht wässrige Lösungen werden im Wesentlichen Lösungen des Wirkstoffes in absolutem Ethanol verwendet. Diese ethanolischen Konzentrate müssen ständig bei tiefen Temperaturen von 2 bis 8 °C gelagert werden. Die Aufrechterhaltung der Kühlkette und der Nachweis ihrer Einhaltung stellen für die Hersteller und Anwender der Produkte einen erheblichen Aufwand dar und verursachen hohe Kosten. Trotzdem beträgt die Verwendungsfrist der Zubereitungen typischerweise nur 18 Monate. Handelsbezeichnungen dieser Produkte sind z.B. Pridax® (Gebro, Pharmore), Alprestil® (Gebro), Miniprog® (Pfizer, Pharmacia) oder Alprostadil "PINT"® (Pint Pharma).

In der Literatur sind auch ölige und liposomale Zubereitungen und Konzentrate beschrieben. Letztere werden in Japan z.B. unter den Markenbezeichnungen Liple® (Tanabe) und Palux® (Taisho) auch vermarktet.

Die Stabilität halbfester Zubereitungen ist auch unter günstigsten Bedingungen auf wenige Monate beschränkt, was den praktischen Einsatz solcher Produkte insbesondere in der Indikation der erektilen Dysfunktion stark limitiert. Als topische Zubereitungen werden reine Lipogele beschrieben, die natürliche Öle, Fettsäuren, Fettalkohole oder synthetische Lipide, wie z.B. Isopropylmyristat, enthalten und die zusätzlich mit Verdickungsmitteln, wie z.B. Poloxameren, verdickt werden können. Beschrieben sind derartige Lipogel z.B. in KR 20010011625 und in JP 0383925.

Weitere Darreichungsformen, z.B. feste Stäbchen zur Anwendung in der Harnröhre werden z.B. unter dem Markennamen Muse® von der Firma Abbott angeboten.

Für die vorliegende Erfindung stellt die Lösung des Wirkstoffes in absolutem Ethanol (z.B. Pridax® 20 µg/ml Konzentrat zur Herstellung einer Infusionslösung) den nächstkommenden Stand der Technik dar. Der Hauptvorteil der ethanolischen Lösung besteht darin, dass sie einfacher zu einer infusionsfertigen Lösung verarbeitet werden kann als ein Lyophilisat. Das Lyophilisat muss zunächst in einem ersten Schritt im Vial mit einem geeigneten Lösungsmittel rekonstituiert werden; anschließend muss diese Lösung in einem zweiten Schritt der Infusionslösung zugesetzt werden. Demgegenüber kann die Ampulle direkt in einem Schritt zu einer Infusionslösung gegeben werden. Diese Vereinfachung des Handlings reduziert auch das Risiko von Manipulationsfehlern und das Risiko, Keime einzuschleppen.

Ein weiterer Vorteil der ethanolischen Lösung ist die sehr einfache Zusammensetzung der Formulierung, bestehend ausschließlich aus dem Wirkstoff und dem Lösungsmittel. Sie ermöglicht eine im Vergleich zu einem Lyophilisat viel einfachere und preisgünstigere Herstellung.

Ein weiterer Vorteil ist, dass die einfach Zusammensetzung der Formulierung die Analytik der Zubereitung wesentlich erleichtert. Wegen der geringen Dosierung des Wirkstoffes müssen im Rahmen der Qualitätskontrolle Verunreinigungen bis in einen Konzentrationsbereich von 10 ng/ml quantifiziert werden. Bei so niedrigen Konzentrationen ist es ein großer Vorteil, wenn die Zubereitung ohne Aufarbeitung und ohne Abtrennung von Hilfsstoffen direkt analysierbar ist.

Ein Nachteil der ethanolischen Lösung besteht in ihrer geringen chemischen Stabilität. Sie hat folgende konkrete nachteilige Auswirkungen:
- Es müssen hohe Konzentrationen von Abbauprodukten im Fertigprodukt in Kauf genommen werden (z.B. 6 % PGA1)
- Die Laufzeit der Fertigprodukte ist begrenzt (z.B. 18 Mon)
- Die Lagerung der Fertigprodukte muss kühl erfolgen, eine permanente Kühlkette ist aufrecht zu erhalten (2 - 8 °C)
- Die Standzeit bei Raumtemperatur, die im Zuge des Herstellprozesses unvermeidlich ist, muss auf wenige Stunden begrenzt werden.

Es besteht deshalb ein hohes Interesse daran, eine, vorzugsweise ethanolische, Alprostadil Lösung zu schaffen, die weiterhin einfach in der Anwendung ist, die einfach und kostengünstig hergestellt werden kann und die unkompliziert zu analysieren ist, die aber gleichzeitig eine verbesserte Stabilität aufweist.

Aufgabe der Erfindung ist es deshalb, eine solche Formulierung zu entwickeln, die zusätzlich zu den bisherigen Vorteilen der ethanolischen Lösung (z.B. von Pridax®) eine geringere Konzentration an Abbauprodukten enthält, eine verbesserte Stabilität und eine längere Haltbarkeit aufweist und ein weniger strenges Kühlregime bei der Herstellung, beim Transport und bei Lagerung erfordert.

Diese Aufgabe wird dadurch gelöst, dass die Lösung von Alprostadil eine geringe Menge einer sauren Komponente enthält.

Erfindungsgemäß liegt also eine flüssige pharmazeutische Zusammensetzung vor, insbesondere als Konzentrat zur weiteren Herstellung einer Infusionslösung, enthaltend Alprostadil als Wirkstoff, gelöst in zumindest einem nicht-wässrigen Lösungsmittel, enthaltend zumindest eine Säure bzw. eine als Säure bzw. Protonendonator wirkende Substanz als Stabilisator für den Wirkstoff.

Überraschenderweise wurde gefunden, dass die erfindungsgemäße Lösung im Vergleich zum Stand der Technik (Pridax® 20 µg/ml Konzentrat zur Herstellung einer Infusionslösung) eine wesentlich bessere Lagerstabilität aufweist, insbesondere, dass die Bildung des Hauptabbauproduktes PGA1 deutlich verlangsamt ist. Dieser Stabilisierungseffekt ist so ausgeprägt, dass die erfindungsgemäße Zubereitung geeignet ist, alle Zielsetzungen der Erfindung zu erreichen. Die obere Spezifikationsgrenze für das Abbauprodukt PGA1 kann reduziert werden kann (z.B. von 6 % auf 4 %). Die Haltbarkeit des Produktes wird verlängert (z.B. von 18 Monate auf 36 Monate). Die Standzeit der Lösung während der Herstellung bei Raumtemperatur wird erhöht (z.B. von 4 auf 8 h), wodurch auch die Chargengröße erhöht werden kann. Das analytische Verfahren für die Zubereitung wird durch den Zusatz der sauren Komponente nicht komplizierter.

Aus der Literatur (Vieillard et al., Pharmaceut Anal Acta 2013, 4:4, 4.00-1488 Alprostadil Kommentar zur Ph. Eur. 4.00) ist bekannt, dass der Wirkstoff Alprostadil sehr empfindlich gegenüber Basen, Säuren und Oxidationsmitteln ist, wobei insbesondere Basen und Säuren die Abspaltung von Wasser zum Hauptabbauprodukt PGA1 beschleunigen. Wegen der geringen Dosierung des Alprostadils in der Konzentratlösung von 20 µg/ml bis 500 µg/ml, ist damit zu rechnen, dass bereits kleine Mengen der oben genannten Verunreinigungen zu einer Verschlechterung der Stabilität führen. Wegen ihres ubiquitären Vorkommens ist eine konsequente Vermeidung dieser Verunreinigungen mit dem derzeitigen Stand der Technik, allein durch die Prozessführung bzw. die Reinheit von Packmitteln und Lösungsmitteln nicht möglich bzw. würden solche Technologien die Herstellkosten soweit erhöhen, dass das Produkt zu keinem marktüblichen Preis mehr angeboten werden könnte.

Völlig überraschend wurde entgegen der in der Fachliteratur vertretenen Meinung gefunden, dass sehr geringe Mengen von organischen oder anorganischen Säuren nicht zu der zu erwartenden Verschlechterung der Stabilität führen, sondern im Gegenteil zu einer wesentlichen Verbesserung. Unter sehr geringen Mengen von organischen oder anorganischen Säuren sind im Sinne der Erfindung Mengen zu verstehen, die zu einer Säurekonzentration von 500 µg/ml oder kleiner führen.

Dem Stand der Technik entsprechend wird die Konzentration des Wirkstoffes in ethanolischen Alprostadil-Lösungen in der Einheit µg/ml angegeben. In Analogie dazu wird in dieser Erfindung auch die Konzentration der Säure in der Einheit µg/ml angegeben. Eine Umrechnung in die Einheit µg/g kann jederzeit vorgenommen werden, indem durch die Dichte des Lösungsmittels (absoluter Alkohol ca. 0,789 g/ml) dividiert wird. Bei der theoretischen Abschätzung der für eine Stabilisierung benötigten Säuremenge aus der exponentiellen Regression der erzeugten Stabilitätsdaten ergibt sich die Säuremenge in der Einheit µM, entsprechend µmol/l. Mit Hilfe des Molekulargewichts der jeweiligen Säure kann diese Konzentrationsangabe ebenfalls jederzeit in die Einheit µg/ml umgerechnet werden.

In vorteilhaften Formulierungen werden geringe Säurekonzentrationen von 0,05 - 500 µg/ml eingesetzt, vorzugsweise 0,1 - 100 µg/ml, insbesondere 5 - 50 µg/ml. In einer besonders bevorzugten Ausführungsform der Erfindung ist zur Stabilisierung einer ethanolischen Lösung enthaltend 20 µg/ml Alprostadil ein Säurezusatz von ebenfalls 20 µg/ml vorgesehen.

Insbesondere tritt dieser vorteilhafte Effekt auf, wenn Alprostadil in einer Konzentration von 1-1000 µg/ml, vorzugsweise 5-750 µg/ml, insbesondere 20-500 µg/ml, enthalten ist.

Zur analytischen Bestimmung der artifiziell zugesetzten Säure können dem Stand der Technik entsprechende analytische Methoden wie z.B. HPLC, GC, Titration oder spektroskopische Methoden herangezogen werden. Ein Nachweis des Säurezusatzes durch eine Messung des pH-Wertes ist in den erfindungsgemäßen alkoholischen, d.h. nicht wässrigen Formulierungen nicht möglich. In einem vereinfachten Test kann aber die Zugabe von Säure durch Verdünnung des Fertigarzneimittels im Verhältnis von 1:25 mit einer 0,9%igen NaCl Lösung und anschließende Messung des pH-Wertes der beiden Lösungen erfolgen. Eine Senkung des pH-Wertes um mindestens 0.2 Einheiten gegenüber dem Ausgangswert der verwendeten 0,9%igen NaCl Lösung zeigt den Zusatz von Säure in der Formulierung an.

Unter den Begriffen "saure Komponente" und "Säure" werden im Sinne der Erfindung Verbindungen gemäß der Säuredefinition von Brönsted und Lowry verstanden, d.h. Substanzen, welche in der Lage sind Protonen abzugeben bzw. als Protonendonator zu fungieren. Um gegenüber dem Alprostadil als Protonendonator zu wirken, muss eine Säure einen pks-Wert aufweisen, der niedriger als 4,85, dem pks-Wert des Alprostadils, ist. Es kann sich hier sowohl um anorganische als auch organische Substanzen handeln. Die Säuren können sowohl einbasig, als auch mehrbasig sein. Vorteilhaft geeignete Säuren im Sinne der Erfindung sind solche organische und anorganische Säuren, die auf Grund ihres toxikologischen Profils in parenteralen Arzneimitteln einsetzbar sind und die sich in den erfindungsgemäßen Konzentrationen in den verwendeten Lösungsmitteln, insbesondere in absolutem Alkohol, auch bei niedrigen Temperaturen vollständig und klar lösen. Beispiele für solche Säuren sind Salzsäure, Essigsäure, Zitronensäure, Äpfelsäure, Milchsäure, Weinsäure oder Acetylcystein.
Als im Sinne der Erfindung besonders geeignet haben sich organische Carbonsäuren, insbesondere wegen ihres breiten Stabilisierungsoptimums mehrbasige, organische Hydroxycarbonsäuren, erwiesen. Vorzugsweise werden deshalb Säuren wie z.B. Zitronensäure, Äpfelsäure oder Weinsäure eingesetzt, besonders bevorzugt ist die Verwendung von DL-Äpfelsäure.
Verwendbar sind allerdings auch anorganische Säuren, insbesondere ausgewählt aus der Gruppe Salzsäure oder Phosphorsäure.

Stabilitätsversuche haben ergeben, dass sich eine Stabilitätsverbesserung im Wesentlichen mit all denjenigen Säuren erreichen lässt, die die hier angegebenen Bedingungen erfüllen. Eine Stabilisierung lässt sich stets sowohl daran erkennen, dass der Gehalt an Alprostadil während einer Testlagerung langsamer abnimmt, als auch daran, dass der Gehalt an Verunreinigungen, insbesondere an Verunreinigung A langsamer steigt. Die Konzentration, bei der eine maximale Stabilitätsverbesserung auftritt, sowie das Ausmaß der Stabilitätsverbesserung, hängen signifikant von der Art der verwendeten Säure, insbesondere von ihrem pKs1-Wert, ab, wobei die stabilisierende Wirkung in allen Fällen ein Konzentrationsoptimum durchläuft.

Keine Stabilitätsverbesserung konnte beim Zusatz saurer Puffersysteme beobachtet werden. Während z. B. der Zusatz von 50 µg/ml Essigsäure gegenüber einer rein ethanolischen Alprostadil Lösung zu einer Verbesserung der Stabilität führt, wird die Stabilität durch den Zusatz von Puffermischungen bestehend aus 50 µg/ml Essigsäure und 50 µg/ml Natriumacetat oder 25 µg/ml Essigsäure und 25 µg/ml Natriumacetat verschlechtert.

Beim Zusatz reiner Säuren hat sich gezeigt, dass der Konzentrationsbereich der optimalen Stabilisierungswirkung umso tiefer bzw. niedriger liegt und auch umso schmaler bzw. enger ist, je stärker die Säure ist. Die nachfolgende Tabelle 1 macht diesen Zusammenhang für eine Lösung von Alprostadil in wasserfreiem Ethanol in einer Konzentration von 20 µg/ml deutlich:

**Tabelle 1: Für die Stabilisierung einer Lösung von 20 µg/ml Alprostadil in wasserfreiem Ethanol geeignete Säuren und dafür geeignete Säurekonzentrationen**

| Säure | pks1-Wert | Konzentrationsbereich der Stabilisierungswirkung in µg/ml | Konzentrationsbereich der optimalen Stabilisierungswirkung in µg/ml |
|---|---|---|---|
| Salzsäure | -7 | 0,1 - 0,8 | 0,3 - 0,7 |
| Phosphorsäure | + 2,16 | 0,5 - 15 | 1 - 10 |
| Weinsäure | + 2,98 | 2 - 150 | 5 - 50 |
| DL-Äpfelsäure | + 3,46 | 5 - 200 | 10 - 100 |
| Acetylcystein | + 3,82 | 5 - 350 | 10 - 100 |
| Essigsäure | + 4,75 | 20 - 500 | 50 - 250 |

Tabelle 1 enthält somit vorteilhafte Konzentrationen von vorteilhaft einsetzbaren Säuren, bei denen sich experimentell die besten Stabilisierungen für Alprostadil ergeben haben.

Aus den Vergleichsversuchen lässt sich weiters ableiten, dass sich vorteilhafte Stabilisierungseffekte erreichen lassen, wenn die Säure einen pKs1-Wert von < 4,85, insbesondere im Bereich von 2 bis 4,8, aufweist.

In diesem Zusammenhang hat es sich als vorteilhaft erwiesen, wenn die Säure einen pKs1-Wert von größer gleich 0 bis kleiner 4,85 aufweist und in einer Konzentration von größer gleich c min = 1,45* e hoch (1,02* pKs1) µM (=µmol/l) und kleiner gleich c max = 11,75* e hoch (1,35* pKs1) µM (=µmol/l) enthalten ist oder wenn die Säure einen pKs1-Wert von kleiner 0 aufweist und in einer Konzentration von größer gleich c min = 1,0 µM (=µmol/l) und kleiner gleich c max = 15 µM (=µmol/l) enthalten ist.

Dabei hat es sich für die Alprostadil-Stabilisierung weiters als besonders vorteilhaft erwiesen, wenn die anorganische oder organische Säure einen pKs1-Wert von größer gleich 2 bis kleiner gleich 4,8 aufweist und in einer Konzentration von 0,5 bis 500 µg/ml, insbesondere von 1 bis 250 µg/ml, enthalten ist.

Unter dem Begriff "nicht-wässriges Lösungsmittel" werden vorliegend Lösungsmittel verstanden, die überwiegend nicht aus Wasser bestehen bzw. kaum Wasser enthalten. Geringe Mengen bzw. Restmengen an Wasser bis zu einigen Gew.-% können aber enthalten sein.

Dementsprechend weist das nicht-wässrige Lösungsmittel vorteilhafterweise einen maximalen Wassergehalt bis 7,4 Gew.-%, insbesondere bis 4 Gew.-%, vorzugsweise bis 0,1 Gew.-%, auf. Experimentell konnten durch diese Mengen von Wasser keine störenden Verschlechterungen der Stabilität festgestellt werden.
Vorteilhafterweise kann als nicht-wässriges Lösungsmittel Propylenglycol, 1,3 Butandiol, Ethylacetat, Ethanol 96 % PhEur (95.1 - 96.9 % (V/V) entsprechend 92,6 - 95,2 Gew.-%) oder Wasserfreies Ethanol PhEur (min. 99.5 % (V/V) entsprechend min. 99,2 Gew.-%) bzw. absoluter Ethanol eingesetzt werden, jeweils gemäß den Definitionen und Anforderungen der Europäischen Pharmakopöe. Wasserfreies Ethanol PhEur (min. 99.5 % (V/V)) und absoluter Ethanol werden dabei als Synonyme verstanden, oft werden sie auch als 100%iges Ethanol bezeichnet, was vorliegend ebenfalls gleichbedeutend ist.
All diese Lösungsmittel sind in parenteralen Zubereitungen auf Grund ihres toxikologischen Profils anwendbar und der Wirkstoff Alprostadil ist darin in ausreichender Konzentration löslich. Auch Mischungen dieser Lösungsmittel sind vorteilhaft einsetzbar. Darüber hinaus können dem Lösungsmittel auch Lösungsvermittler wie Polyethylenglycol oder Polysorbat zugesetzt sein.

Eine vorteilhafte, weil vor allem besonders einfache, Zusammensetzung liegt vor, wenn die Zusammensetzung abschließend und ausschließlich aus Alprostadil als Wirkstoff, gelöst in zumindest einem nicht-wässrigen Lösungsmittel, und aus zumindest einer Säure bzw. einer als Säure bzw. Protonendonator wirkenden Substanz als Stabilisator für den Wirkstoff besteht und keine weiteren Bestandteile, Exzipienten oder Additive umfasst.

Die Abfüllung der erfindungsgemäßen Lösung erfolgt in geeignete Primärpackmittel. Als Primärpackmittel werden in diesem Zusammenhang Packmittel verstanden, welche in direktem Kontakt mit dem Arzneimittel stehen. Sie müssen einerseits den Anforderungen der einschlägigen Arzneibücher entsprechen und müssen darüber hinaus so ausgewählt werden, dass sie möglichst wenig für die Stabilität des Inhalts schädliche Substanzen, wie z.B. basische Verunreinigungen, abgeben. Geeignete Materialien, aus denen für empfindliche Parenteralia einsetzbare Primärpackmittel bestehen, sind dem Fachmann bekannt. Denkbar sind beispielsweise, aber nicht ausschließlich Röhrenglas oder Hüttenglas der hydrolytischen Klasse I oder der hydrolytischen Klasse II, mit Innenvergütung. Ebenso anwendbar sind beispielsweise, aber nicht ausschließlich Kunststoffe wie COP, COC, PP, PE, HDPE, Teflon, PA.

Bevorzugte Primärbehältnisse sind Glasampullen oder Durchstechflaschen (Vials), bestehend aus Röhrenglas der hydrolytischen Klasse I. Als primäres Verschlussmaterial für

Durchstechflaschen kommen Stopfen aus Brombutyl-Kautschuk oder Chlorbutyl-Kautschuk, vorzugsweise beschichtet, in Frage. Weitere mögliche Primärbehältnisse sind beispielsweise, aber nicht ausschließlich, Karpulen oder Fertigspritzen.

Daraus ergibt sich, dass eine gute Stabilisierung der Zusammensetzung bzw. von Alprostadil bei einer Kit-of-parts-Kombination aus der erfindungsgemäßen Zusammensetzung abgefüllt bzw. enthalten in einer Glasampulle, Glasvial, Karpule oder Fertigspritze, vorliegt.

Die Säure wird im Sinne der Erfindung vorzugsweise während des Herstellprozesses des Fertigarzneimittels dem Lösungsmittel zugegeben. Mögliche Ausführungsvarianten wären, die Säure während des Herstellprozesses des Lösungsmittels oder eines weiteren Hilfsstoffes oder während des Herstellprozesses des Wirkstoffes Alprostadil zuzugeben.

Die erfindungsgemäße pharmazeutische Zusammensetzung ist vorteilhafterweise ein Konzentrat für die Erstellung einer parenteral zu verabreichenden Infusionslösung, welches Konzentrat, insbesondere durch einen Verdünnungsschritt, zu der gebrauchsfertigen Infusionslösung für die parenterale Verabreichung herrichtbar oder hergerichtet ist. Durch die Verdünnung, z.B. mit isotonisierter, wässriger Lösung, vorzugsweise mit physiologischer Kochsalzlösung, insbesondere in einem Verhältnis von 1:10 bis 1:500, vorzugsweise 1:20 bis 1:250, ist dann die gebrauchsfertige parenterale Infusionslösung erhältlich.
Alternativ ist die Zusammensetzung als konzentrierte Injektionslösung für die direkte Verabreichung möglich.

Sämtliche in dieser Anmeldung angegebenen Konzentrationsangaben, z.B. für die Säure, für Alprostadil etc, beziehen sind jedenfalls auf ein solches Konzentrat und nicht auf die gebrauchsfertige, verdünnte Infusionslösung.

Erfindungsgemäß ist dementsprechend in einem weiteren Aspekt ein Verfahren zur Herstellung einer flüssigen pharmazeutischen Zusammensetzung, als Konzentrat, vorgesehen, im Zuge dessen dem in einem, wie zuvor beschriebenen, nicht-wässrigen Lösungsmittel gelösten Wirkstoff Alprostadil, zumindest eine Säure bzw. eine als Säure bzw. Protonendonator wirkende Substanz, wie zuvor beschrieben, als Stabilisator für den Wirkstoff zugesetzt wird.

Erfindungsgemäß ist weiters die Verwendung zumindest einer Säure bzw. einer als Säure bzw. Protonendonator wirkenden Substanz, wie zuvor beschrieben, als Stabilisator für den Wirkstoff Alprostadil bzw. für eine, wie zuvor beschriebene, flüssige pharmazeutische Zusammensetzung, enthaltend den in einem nicht-wässrigen Lösungsmittel gelösten Wirkstoff Alprostadil.

Die erfindungsgemäße Zusammensetzung dient in ihrer wichtigsten Indikation zur Behandlung der chronischen arteriellen Verschlusskrankheit im Stadium III und IV, wenn eine Therapie zur Erweiterung der Blutgefäße nicht möglich oder erfolglos ist.

### Beispiele:

Im Folgenden wird die Erfindung an Hand von einigen exemplarischen Ausführungsbeispielen dargestellt:

### Beispiel 1:

### (Laboransatz/Ethanol/2 µg/ml Alprostadil/40 µg/ml Essigsäure/Vials)

In einem 2,5 l Glasreaktor mit Rührwerk wird Ethanol absolut vorgelegt und Essigsäure wasserfrei zugegeben. Nach vollständigem Auflösen wird der Wirkstoff Alprostadil unter Rühren zugegeben, die Lösung in 2ml Typ I Braunglas Vials abgefüllt und mit einem Chlorbutyl-Kautschuk Stopfen verschlossen.

| | Menge / g |
|---|---|
| Alprostadil | 0,043 |
| Ethanol absolut | 1700 |
| Essigsäure, wasserfrei | 0,086 |

### Beispiel 2:

### (Laboransatz / Ethanol / 20 µg/ml Alprostadil / 20 µg/ml Äpfelsäure / Vials)

In einem 2,5 I Glasreaktor mit Rührwerk wird Ethanol absolut vorgelegt und DL-Äpfelsäure zugegeben. Nach vollständigem Auflösen wird der Wirkstoff Alprostadil unter Rühren zugegeben, die Lösung in 2ml Typ I Braunglas Vials abgefüllt und mit einem Chlorbutyl-Kautschuk Stopfen verschlossen.

| | Menge / g |
|---|---|
| Alprostadil | 0,043 |
| Ethanol absolut | 1700 |
| DL-Äpfelsäure | 0,043 |

### Beispiel 3:

### (Laboransatz / Ethanol / 20 µg/ml Alprostadil / 20 µg/ml Äpfelsäure / Fertigspritzen)

In einem 2,5 l Glasreaktor mit Rührwerk wird Ethanol absolut vorgelegt und DL-Äpfelsäure zugegeben. Nach vollständigem Auflösen wird der Wirkstoff Alprostadil unter Rühren zugegeben und die Lösung in Fertigspritzen abgefüllt.

| | Menge / g |
|---|---|
| Alprostadil | 0,043 |
| Ethanol absolut | 1700 |
| DL-Äpfelsäure | 0,043 |

### Beispiel 4:

### (Produktionsansatz / Ethanol / 20 µg/ml Alprostadil / 20 µg/ml DL-Äpfelsäure / Ampullen)

Die Formulierung gemäß Beispiel 4 stellt eine besonders bevorzugte Ausführungsform der Erfindung dar.

In einem 20 l Glasreaktor mit Rührwerk wird gekühlter Ethanol absolut vorgelegt und DL-Äpfelsäure zugegeben. Nach vollständigem Auflösen wird der Wirkstoff Alprostadil unter Rühren zugegeben. Nach vollständigem Auflösen des Wirkstoffes wird die Lösung über einen Sterilfilter mit einer Porenweite von 0,2 µm filtriert und unter aseptischen Bedingungen in 2ml Typ I Braunglas Ampullen abgefüllt. Die abgefüllten Ampullen werden auf sichtbare Partikel und Dichtigkeit überprüft, etikettiert und sekundärverpackt.

| | Menge / g |
|---|---|
| Alprostadil | 0,40 |
| Ethanol absolut | 15780 |
| DL-Äpfelsäure | 0,40 |

Weitere Ausführungsbeispiele werden in den nachfolgenden Tabellen 2 und 3 aufgeführt, wobei jeweils nur die Zusätze zum Lösungsmittel in der Einheit µg angegeben werden. Alle in den Tabellen aufgeführten Beispiele können in beliebiger Chargengröße hergestellt und in alle Arten geeigneter Packmittel abgefüllt werden.

**Tabelle 2: Rezepturbeispiele für Zubereitungen in der Alprostadil-Konzentration 20 µg/ml**

| Beispiel | 5 | 6 | 7 | 8 | 9 | 10 | 11 | 12 | 13 | 14 |
|---|---|---|---|---|---|---|---|---|---|---|
| Ethanol absolut | ad 1ml | ad 1ml | ad 1ml | ad 1ml | ad 1ml | ad 1ml | ad 1ml | ad 1ml | | |
| Ethanol 96 % | | | | | | | | | ad 1ml | |
| Propylenglycol | | | | | | | | | | ad 1ml |
| | | | | | | | | | | |
| PGE 1 | 20 | 20 | 20 | 20 | 20 | 20 | 20 | 20 | 20 | 20 |
| | | | | | | | | | | |
| Essigsäure | | | | | | | | 100 | | |
| HCl | | | | | | 0,5 | | | | |
| Phosphorsäure | | | | | | | 5 | | | |
| Äpfelsäure | | 50 | | 10 | | | | | 50 | 20 |
| Acetylcystein | 20 | | | 10 | | | | | | |
| Zitronensäure | | | | | 20 | | | | | |
| Weinsäure | | | 15 | 10 | | | | | | |

**Tabelle 3: Rezepturbeispiele für Zubereitungen in Alprostadil-Konzentrationen 10-1000 µg/ml**

| Beispiel | 15 | 16 | 17 | 18 | 19 | 20 | 21 | 22 | 23 |
|---|---|---|---|---|---|---|---|---|---|
| Ethanol absolut | ad 1ml | ad 1ml | ad 1ml | ad 1ml | ad 1ml | ad 1ml | ad 1ml | | |
| Ethanol 96 % | | | | | | | | ad 1ml | |
| Propylenglycol | | | | | | | | | ad 1ml |
| | | | | | | | | | |
| PGE 1 | 10 | 10 | 500 | 500 | 500 | 1000 | 1000 | 500 | 500 |
| | | | | | | | | | |
| Essigsäure | 40 | | | | | | | | 100 |
| HCl | | | | | 0,25 | 0,75 | | | |
| Phosphorsäure | | | | | | | 7,5 | | |
| Äpfelsäure | | 20 | 20 | 20 | | | | 50 | |
| Acetylcystein | | | | 20 | | | | | |

### Nachweis der stabilisierenden Wirkung des Säurezusatzes:

Der Nachweis der stabilisierenden Wirkung des Säurezusatzes wurde an Hand von Stabilitätsprüfungen bei der vorgeschriebenen Lagertemperatur von 4 °C und unter Stressbedingungen bei 25 °C /65% RH geführt. Als Packmittel wurden jeweils 2 ml Vials aus Röhrenglas der hydrolytischen Klasse I verwendet. Diese Glasqualität ist identisch mit der Glasqualität für Ampullen. Nachfolgend wird nur über die Ergebnisse der Tests bei 25 °C berichtet, da diese Tests bereits nach kürzerer Lagerdauer Aussagen zu Unterschieden in der Stabilität erlauben. Aus langjährigen Stabilitätsprüfungen des Handelsproduktes Pridax® 20 (Pridax 20 µg/ml Konzentrat zur Herstellung einer Infusionslösung) ist bekannt, dass die Abbaureaktionen in der Zubereitung bei 4 °C qualitativ in der gleichen Weise ablaufen wie bei 25 °C, allerdings etwa um den Faktor 10 langsamer.

In einem ersten, diese Erfindung konstituierenden Versuch wurde als Kontrolle die Formulierung des Handelsproduktes Pridax® 20 µg/ml Konzentrat zur Herstellung einer Infusionslösung (Musterbezeichnung X03 130002-3), die als nächstkommender Stand der Technik zu betrachten ist, gegen eine erfindungsgemäße Testformulierung, enthaltend 20 µg/ml Essigsäure und mit ansonsten gleicher Zusammensetzung (Musterbezeichnung X03 130002-9), geprüft.

Der Gehalt an PGE1 wird dabei in der Einheit (µg/ml) gemessen. In allen nachfolgenden Ergebnistabellen wird der Gehalt zur besseren Vergleichbarkeit in % des Ausgangswertes von PGE1 angegeben. Die Konzentration des Abbauproduktes PGA1 wird in Massenprozent (% m/m) bezogen auf den deklarierten Gehalt an PGE1 gemessen und angegeben.

Die nachfolgende Tabelle 4 zeigt für beide Formulierungen die Gehalte an PGE1 (Wirkstoff) und PGA1 (Abbauprodukt), jeweils direkt nach Herstellung (t = 0) und nach einer Lagerung über 12 Wochen bei 25° C / 65% RH (t = 12 w). Die Absolutwerte von PGE1 direkt nach Herstellung betrugen 18,3 µg/ml für die säurefreie Formulierung und 18,4 µg/ml für die essigsäurehaltige Formulierung.

**Tabelle 4:**

| Kontrolle vs 20 µg/ml Essigsäure, Lagerung 12 Wo bei 25° C | | | |
|---|---|---|---|
| Formulierung | | t = 0 | t = 12 Wochen |
| X03 130002-3 (ohne Säure) | PGE1 | 100 % | 84,2% |
| | PGA1 | 0,28 % | 19,8% |
| X03 130002-9 (mit 20 µg/ml Essigsäure) | PGE1 | 100 % | 95,6% |
| | PGA1 | 0,13 % | 5,22% |

Der Zusatz von 20 µg/ml Essigsäure zu der Alprostadil Lösung gemäß dem Stand der Technik führt nach 12 Wochen Lagerung bei 25 °C zueinem deutlichen Stabilisierungseffekt, erkennbar an einer signifikant geringeren Abnahme des Gehalts an PGE1 (Alprostadil Wirkstoff) und an einer signifikant geringeren Zunahme des Gehalts an PGA1 (Alprostadil Abbauprodukt).

Nachweis Einfluss der Menge bzw. Konzentration des Säurezusatzes:
In einem weiteren Versuch wurde der Einfluss der Menge an Essigsäure auf den gefundenen Stabilisierungseffekt untersucht. Dazu wurde die Formulierung des Handelsproduktes Pridax® 20 µg/ml Konzentrat zur Herstellung einer Infusionslösung (Musterbezeichnung X03 130007) gegen Testformulierungen, enthaltend 5 µg/ml, 20 µg/ml und 50 µg/ml Essigsäure und mit ansonsten gleicher Zusammensetzung (Musterbezeichnungen X03 130007-1, X03 130007-2 und X03 130007-1), geprüft.

Die nachfolgende Tabelle 5 sowie die dazugehörige Graphik 1 zeigen für diese vier Formulierungen die Gehalte an PGE1 (Wirkstoff) und PGA1 (Abbauprodukt), jeweils direkt nach Herstellung (t = 0) und nach einer Lagerung über 6 Wochen bei 25° C / 65% RH (t = 6 w). Die Absolutwerte von PGE1 direkt nach Herstellung betrugen zwischen 20,0 und 20,2 µg/ml.

**Tabelle 5:**

| Kontrolle vs Essigsäure in verschiedenen Konzentrationen, Lagerung 6 Wo bei 25° C | | | |
|---|---|---|---|
| Formulierung | | t = 0 | t = 6 Wochen |
| X03 130007 (ohne Säure) | PGE1 | 100% | 93,5 % |
| | PGA1 | 0,13% | 5,15% |
| X03 130007-1 (mit 5 µg/ml Essigsäure) | PGE1 | 100% | 95,0% |
| | PGA1 | 0,12 % | 6,82% |
| X03 130007-2 (mit 20 µg/ml Essigsäure) | PGE1 | 100 % | 97,0% |
| | PGA1 | 0,10 % | 3,95 % |
| X03 130007-3 (mit 50 µg/ml Essigsäure) | PGE1 | 100 % | 99,0% |
| | PGA1 | 0,11 % | 2,36% |

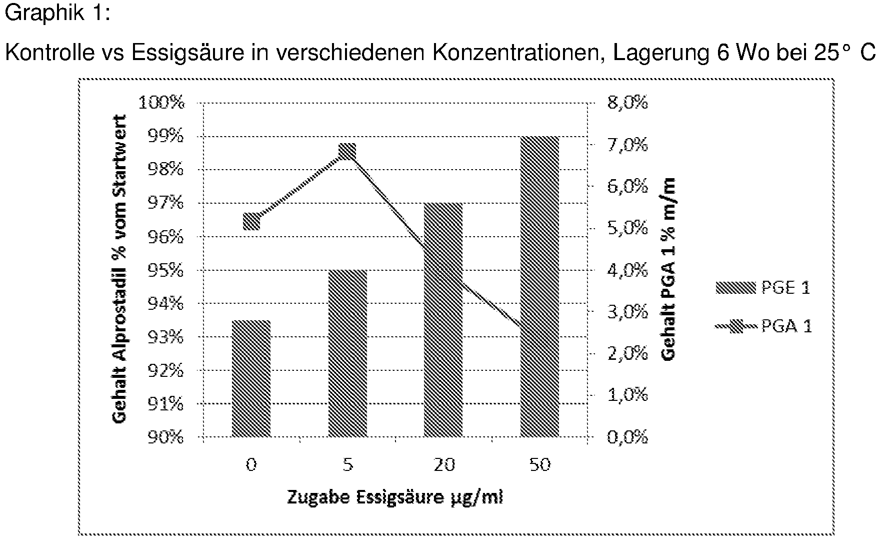

Die stabilisierende Wirkung des Zusatzes 20 µg/ml Essigsäure zu der Alprostadil Lösung konnte in diesem Versuch mittels der Probe X03 130007-2 reproduziert werden. Die Erhöhung der Essigsäuremenge auf 50 µg/ml führte zu einer weiteren Verbesserung der Stabilität, bei einer Konzentration von nur 5 µg/ml konnte kein signifikanter Stabilisierungseffekt beobachtet werden.

### Nachweis Art der Säure:

In einem weiteren Versuch wurde die stabilisierende Wirkung verschiedener Säuren bei einem konstanten Säurezusatz von jeweils Zusatzes 20 µg/ml vergleichend untersucht. Dazu wurde die Formulierung des Handelsproduktes Pridax® 20 µg/ml Konzentrat zur Herstellung einer Infusionslösung (Musterbezeichnung X03 130007) gegen Testformulierungen, enthaltend jeweils 20 µg/ml Essigsäure (Musterbezeichnung X03 130007-2), Äpfelsäure (Musterbezeichnung X03 130007-5), Weinsäure (Musterbezeichnung X03 130007-8), Zitronensäure (Musterbezeichnung X03 130007-11), Acetylcystein (Musterbezeichnung X03 130007-14), Phosphorsäure (Musterbezeichnung X03 140006-6) und Salzsäure (Musterbezeichnung X03 140006-3) und mit ansonsten gleicher Zusammensetzung geprüft.

Die nachfolgende Tabelle 6 zeigt für diese acht Formulierungen die Gehalte an PGE1 (Wirkstoff) und PGA1 (Abbauprodukt), jeweils direkt nach Herstellung (t = 0) und nach einer Lagerung über 6 Wochen bei 25° C / 65% RH (t = 6 w). Die Absolutwerte von PGE1 direkt nach Herstellung betrugen zwischen 20,0 und 20,3 µg/ml.

**Tabelle 6:**

| Kontrolle vs 20 µg/ml verschiedener Säuren, Lagerung 6 Wo bei 25° C | | | |
|---|---|---|---|
| Formulierung | | t = 0 | t = 6 Wochen |
| X03 130007 (ohne Säure) | PGE1 | 100 % | 93,5 % |
| | PGA1 | 0,13 % | 5,15% |
| X03 130007-2 (mit 20 µg/ml Essigsäure) | PGE1 | 100 % | 97,0% |
| | PGA1 | 0,10% | 3,95 % |
| X03 130007-5 (mit 20 µg/ml Äpfelsäure) | PGE1 | 100 % | 100,0% |
| | PGA1 | < 0,05 % | 0,60% |
| X03 130007-8 (mit 20 µg/ml Weinsäure) | PGE1 | 100 % | 98,0% |
| | PGA1 | 0,06% | 0,67 % |
| X03 130007-11 (mit 20 µg/ml Zitronensäure) | PGE1 | 100 % | 99,0% |
| | PGA1 | 0,09 % | 0,50% |
| X03 130007-14 | PGE1 | 100 % | 98,0% |
| (mit 20 µg/ml Acetylcystein) | PGA1 | < 0,05 % | 0,71 % |
| X03 140006-6 (mit 20 µg/ml Phosphorsäure) | PGE1 | 100 % | 92,0% |
| | PGA1 | 0,06% | 13,56% |
| X03 140006-3 (mit 20 µg/ml Salzsäure) | PGE1 | 100 % | 0% |
| | PGA1 | < 0,05 % | < 0,05 |

Die dazugehörige Graphik 2 zeigt für die fünf Formulierungen, an denen eine stabilisierende Wirkung beobachtet wurde, und für die säurefreie Formulierung die Gehalte an PGE1 (Wirkstoff) und PGA1 (Abbauprodukt) nach einer Lagerung über 6 Wochen bei 25° C / 65% RH. Nicht dargestellt sind die Werte der Formulierungen mit Salzsäure und Phosphorsäure, da hier keine Stabilisierung beobachtet wurde. Der Zusatz von 20 µg/ml Salzsäure führt zu einem Totalabbau von Alprostadil und seinem Abbauprodukt PGA1, der Zusatz von 20 µg/ml Phosphorsäure zu einer Verschlechterung der Stabilität im Vergleich zu der säurefreien Formulierung. In beiden Fällen ist mit 20 µg/ml die für die Stabilisierung optimale Konzentration überschritten. Für Essigsäure beginnt die optimale Konzentration etwas höher, ab etwa 50 µg/ml.

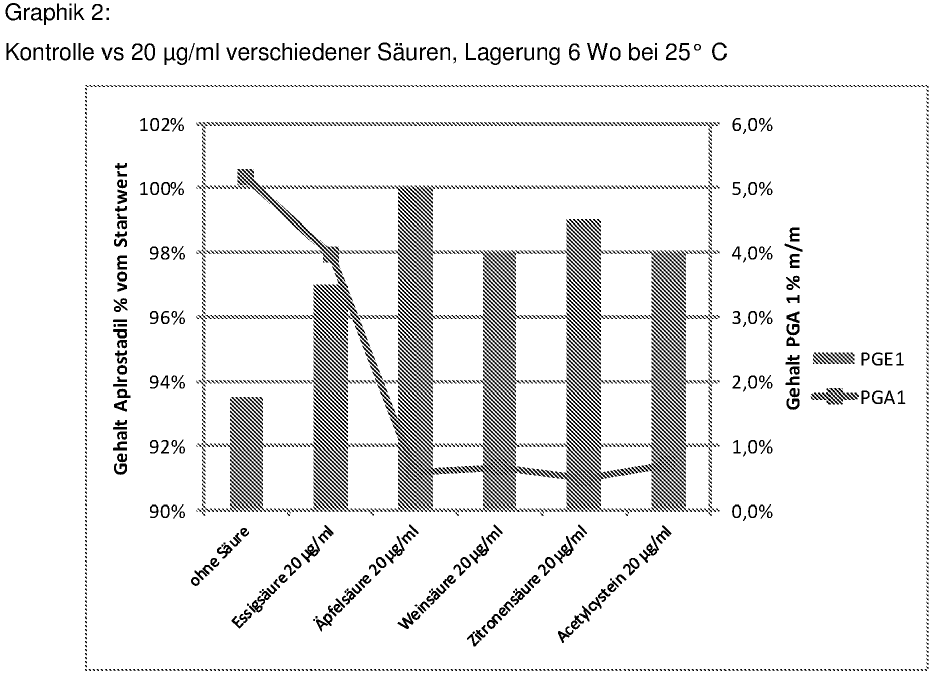

Der Versuch zeigt, dass der stabilisierende Effekt des Säurezusatzes in seinem Ausmaß von der Art der verwendeten Säure abhängt. Die zunächst verwendete Essigsäure hat einen geringeren Stabilisierungseffekt als mehrwertige organische Säuren und Acetylcystein. Für den Funktionsmechanismus des Stabilisierungseffekts bedeutet das, dass es sich nicht nur um eine unspezifische Optimierung der Säurekonzentration, analog der pH-Wert Optimierung in einem wässrigen System, handelt, sondern dass überraschenderweise ein säurespezifischer Stabilisierungsmechanismus zu beobachten ist.

Nachweis eines stabilisierenden Effektes auch der starken Säuren Phosphorsäure und Salzsäure:
Da für Phosphorsäure und Salzsäure bei einer Konzentration von 20 µg/ml keine stabilisierender Effekt auftrat, wurde in weiteren Versuchen geprüft, ob ein solcher Effekt für diese Säuren bei niedrigeren Konzentrationen nachweisbar ist.

Dazu wurde Phosphorsäure zusätzlich in einer Konzentration von 5 µg/ml geprüft. Die nachfolgende Tabelle zeigt, dass bei dieser Konzentration ein guter Stabilisierungseffekt auftritt:

**Tabelle 7:**

| Kontrolle vs 5 und 20 µg/ml Phosphorsäure, Lagerung 6 Wo bei 25° C | | | |
|---|---|---|---|
| Formulierung | | t = 0 | t = 6 Wochen |
| X03 130007 (ohne Säure) | PGE1 | 100 % | 93,5 % |
| | PGA1 | 0,13 % | 5,15% |
| X03 140006-5 (mit 5 µg/ml Phosphorsäure) | PGE1 | 100 % | 98,5 % |
| | PGA1 | 0,08% | 1,96% |
| X03 140006-6 (mit 20 µg/ml Phosphorsäure) | PGE1 | 100 % | 92,0% |
| | PGA1 | 0,06% | 13,56% |

Weiters wurde Salzsäure zusätzlich im Konzentrationsbereich von 0,05 µg/ml bis 2 µg/ml geprüft. Auch bei Salzsäure wurde ein stabilisierender Effekt in einem Konzentrationsbereich von 0,1 - 0,8 µg/ml beobachtet, das Optimum der Stabilisierung wurde bei etwa 0,5 µg/ml beobachtet, bereits bei einem Zusatz von 2 µg/ml war die Stabilität signifikant schlechter als ohne Säurezusatz.

### Nachweis Menge bzw. Konzentration an Äpfelsäure:

Da sich beim Vergleich der unterschiedlichen Säuren Äpfelsäure als besonders tauglich erwies und da Äpfelsäure wegen ihre toxikologischen Profils für die Verwendung in parenteralen Zubereitungen sehr gut geeignet ist, wurde in einem weiteren Versuch der Einfluss der Menge bzw. der Konzentration an Äpfelsäure auf den gefundenen Stabilisierungseffekt untersucht. Dazu wurde - analog zum oben mit Essigsäure beschriebenen Versuch - die Formulierung des Handelsproduktes Pridax® 20 µg/ml Konzentrat zur Herstellung einer Infusionslösung (Musterbezeichnung X03 130007) gegen Testformulierungen, enthaltend 5 µg/ml, 20 µg/ml und 50 µg/ml DL-Äpfelsäure und mit ansonsten gleicher Zusammensetzung (Musterbezeichnungen X03 130007-4, X03 130007-5 und X03 130006-1), geprüft.

Die nachfolgende Tabelle 8 sowie die dazugehörige Graphik 3 zeigen für diese vier Formulierungen die Gehalte an PGE1 (Wirkstoff) und PGA1 (Abbauprodukt), jeweils direkt nach Herstellung (t = 0) und nach einer Lagerung über 6 Wochen bei 25° C / 65% RH (t = 6 w). Die Absolutwerte von PGE1 direkt nach Herstellung betrugen zwischen 20,0 und 20,1 µg/ml.

**Tabelle 8:**

| Kontrolle vs Äpfelsäure in verschiedenen Konzentrationen, Lagerung 6 Wo bei 25° C | | | |
|---|---|---|---|
| Formulierung | | t = 0 | t = 6 Wochen |
| X03 130007 (ohne Säure) | PGE1 | 100 % | 93,5 % |
| | PGA1 | 0,13 % | 5,15% |
| X03 130007-4 (mit 5 µg/ml DL-Äpfelsäure) | PGE1 | 100 % | 100,0% |
| | PGA1 | < 0,05 % | 1,19 % |
| X03 130007-5 (mit 20 µg/ml DL-Äpfelsäure) | PGE1 | 100 % | 100,0% |
| | PGA1 | < 0,05 % | 0,60% |
| X03 130007-6 (mit 50 µg/ml DL-Äpfelsäure) | PGE1 | 100 % | 98,5 % |
| | PGA1 | < 0,05 % | 0,72% |

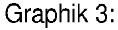

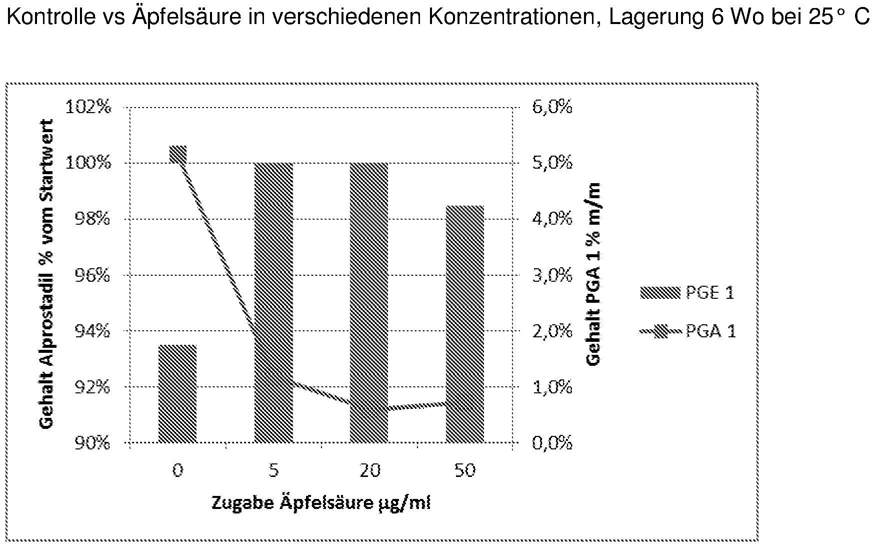

Der Versuch zeigt, dass Äpfelsäure, im Gegensatz zu Essigsäure das Alprostadil schon bei dem sehr geringen Zusatz von 5 µg/ml signifikant stabilisiert. Eine Erhöhung des Säurezusatzes auf 20 µg/ml führt zu einer Verbesserung des Stabilisierungseffekts, eine weitere Erhöhung auf 50 µg/ml führt dagegen, wiederum im Gegensatz zu Essigsäure, zu keiner weiteren Verbesserung der Stabilität.

Zusammenfassung der Versuchsergebnisse zur Art und zur Menge der benötigten Säure:
Geeignet zur Stabilisierung von Alprostadil in alkoholischer Lösung sind solche Säuren, deren pKs1-Wert niedriger als der pKs-Wert von Alprostadil selbst, also niedriger als 4,85 ist,
und die außer ihrer Acidität keine weiteren die Stabilität beeinflussenden Eigenschaften haben.

Für die geeigneten Säuren besteht ein Zusammenhang zwischen dem pKs1-Wert und der benötigten Säuremenge bzw. -konzentration in der Weise, dass bei sinkendem pKs1-Wert auch der Säurebedarf sinkt.

Aus den Stabilitätswerten kann durch exponentielle Regression annähernd folgender Zusammenhang zwischen dem pKs1-Wert der stabilisierenden Säure und der benötigten Säurekonzentration hergestellt werden:
C max in µM c= 11,75 e(1,35* pKs1)
C min in µM c = 1,45 e(1,02* pKs1)

Diese Formeln geben die zur Stabilisierung benötigte Säurekonzentration in der Einheit µM (= µmol / l) an. Sie gilt für geeignete organische und anorganische Säuren in einem pKs1-Bereich von 0 bis 4,85.

Für Säuren mit einem pKs1 < 0 wird dieser mit 0 angenommen, woraus sich folgende konstante Werte ergeben:
C max = 13,2 µM
C min = 1,3 µM

Die zuvor bereits beschriebene Tabelle 1 gibt beispielhaft zur Stabilisierung geeignete Konzentrationen für verschieden Säuren in µg/ml an.

## Patentansprüche

1. Flüssige pharmazeutische Zusammensetzung als Konzentrat zur Herstellung einer Infusionslösung, bestehend aus Alprostadil als Wirkstoff, gelöst in einem nicht-wässrigen Lösungsmittel,
wobei das nicht-wässrige Lösungsmittel Propylenglycol, 1,3 Butandiol, Ethylacetat, Ethanol 96 % PhEur (95.1 - 96.9 % (V/V)) oder Wasserfreies Ethanol PhEur (min. 99.5 % (V/V)) bzw. absoluter Ethanol oder Mischungen davon ist,
und aus zumindest einer Säure bzw. einer als Säure bzw. Protonendonator wirkenden Substanz als Stabilisator für den Wirkstoff,
wobei die Säure einen pKs1-Wert von < 4,85 aufweist.

2. Zusammensetzung nach Anspruch 1, **dadurch gekennzeichnet, dass** die Säure eine organische Säure, insbesondere eine Carbonsäure, ist, und/oder dass die Säure eine Hydroxycarbonsäure ist.

3. Zusammensetzung nach einem der Ansprüche 1 bis 2, **dadurch gekennzeichnet, dass** die Säure aus der Gruppe Äpfelsäure, Essigsäure, Weinsäure, Milchsäure, Acetylcystein oder Zitronensäure ausgewählt ist, und/oder dass die Säure eine mehrwertige Hydroxycarbonsäure, vorzugsweise Zitronensäure, Äpfelsäure oder Weinsäure, ist.

4. Zusammensetzung nach einem der Ansprüche 1 bis 3, **dadurch gekennzeichnet, dass** die Säure Acetylcystein ist, und/oder dass die Säure Äpfelsäure, insbesondere DL- Äpfelsäure, ist.

5. Zusammensetzung nach Anspruch 1, **dadurch gekennzeichnet, dass** die Säure eine anorganische Säure, insbesondere ausgewählt aus der Gruppe Salzsäure oder Phosphorsäure, ist.

6. Zusammensetzung nach einem der Ansprüche 1 bis 5, **dadurch gekennzeichnet, dass** die Säure einen pKs1-Wert im Bereich von 2 bis 4,8 aufweist.

7. Zusammensetzung nach einem der Ansprüche 1 bis 6, **dadurch gekennzeichnet, dass** die Säure einen pKs1-Wert von größer gleich 0 bis kleiner 4,85 aufweist und in einer Konzentration von größer gleich c min = 1,45 * e^{(1,02* pKs1)} µM (=µmol/l) und kleiner gleich c max = 11,75 * e^{(1,35* pKs1)} µM (=*mol/l) enthalten ist,
oder dass die Säure einen pKs1-Wert von kleiner 0 aufweist und in einer Konzentration von größer gleich c min = 1,0 µM (=µmol/l) und kleiner gleich c max = 15 µM (=µmol/l), vorzugsweise größer gleich c min = 1,3 µM (=µmol/l) und kleiner gleich c max = 13,2 µM (=µmol/l), enthalten ist.

8. Zusammensetzung nach einem der Ansprüche 1 bis 7, **dadurch gekennzeichnet, dass** die Säure in einer Konzentration von 0,05-500 µg/ml, vorzugsweise 0,1-100 µg/ml, insbesondere 5-50 µg/ml, enthalten ist.

9. Zusammensetzung nach einem der Ansprüche 1 bis 8, **dadurch gekennzeichnet, dass** die Säure einen pKs1-Wert von größer gleich 2 bis kleiner gleich 4,8 aufweist und in einer Konzentration von 0,5 bis 500 µg/ml, insbesondere von 1 bis 250 µg/ml, enthalten ist.

10. Zusammensetzung nach einem der Ansprüche 1 bis 9, **dadurch gekennzeichnet, dass** die Säure Essigsäure ist und in einer Konzentration zwischen 20 und 500 µg/ml, insbesondere zwischen 50 und 250 µg/ml, enthalten ist,
oder dass die Säure Acetylcystein ist und in einer Konzentration zwischen 5 und 350 µg/ml, insbesondere zwischen 10 und 100 µg/ml, enthalten ist,
oder dass die Säure Äpfelsäure, insbesondere DL- Äpfelsäure, ist und in einer Konzentration zwischen 5 und 200 µg/ml, insbesondere zwischen 10 und 100 µg/ml, enthalten ist,
oder dass die Säure Weinsäure ist und in einer Konzentration zwischen 2 und 150 µg/ml, insbesondere zwischen 5 und 50 µg/ml, enthalten ist.

11. Zusammensetzung nach einem der Ansprüche 1 bis 10, **dadurch gekennzeichnet, dass** die Säure Phosphorsäure ist und in einer Konzentration zwischen 0,5 und 15 µg/ml, insbesondere zwischen 1 und 10 µg/ml, enthalten ist,
oder dass die Säure Salzsäure ist und in einer Konzentration zwischen 0,1 und 0,8 µg/ml, insbesondere zwischen 0,3 und 0,7 µg/ml, enthalten ist.

12. Zusammensetzung nach einem der Ansprüche 1 bis 11, **dadurch gekennzeichnet, dass** Alprostadil in einer Konzentration von 1-1000 µg/ml, vorzugsweise 5-750 µg/ml, insbesondere 20-500 µg/ml, enthalten ist.

13. Zusammensetzung nach einem der Ansprüche 1 bis 12, **dadurch gekennzeichnet, dass** diese in Glasampullen, Glasvials, Karpulen oder Fertigspritzen abgefüllt ist.

14. Kit-of-parts, umfassend die Zusammensetzung nach einem der Ansprüche 1 bis 13, enthalten in einer Glasampulle, Glasvial, Karpule oder Fertigspritze.

15. Zusammensetzung nach einem der Ansprüche 1 bis 13, **dadurch gekennzeichnet, dass** die pharmazeutische Zusammensetzung ein Konzentrat zur Herstellung einer gebrauchsfertigen Infusionslösung für die parenterale Verabreichung, insbesondere durch einen Verdünnungsschritt mit einer geeigneten Trägerlösung, ist oder als Konzentrat zur weiteren Herstellung einer verdünnten, gebrauchsfertigen Infusionslösung für die parenterale Verabreichung hergerichtet ist.

16. Gebrauchsfertige parenterale Zusammensetzung, erhältlich durch Verdünnen der konzentrierten Zusammensetzung nach einem der vorangehenden Ansprüche mit isotonisierter, wässriger Lösung, vorzugsweise mit physiologischer Kochsalzlösung, insbesondere in einem Verhältnis von 1:10 bis 1:500, vorzugsweise 1:20 bis 1:250.

17. Verfahren zur Herstellung einer flüssigen pharmazeutischen Zusammensetzung nach einem der vorangehenden Ansprüche, **dadurch gekennzeichnet, dass** dem in einem nicht-wässrigen Lösungsmittel nach einem der vorangehenden Ansprüche gelösten Wirkstoff Alprostadil zumindest eine Säure bzw. eine als Säure bzw. Protonendonator wirkende Substanz nach einem der vorangehenden Ansprüche als Stabilisator für den Wirkstoff zugesetzt wird.

18. Verwendung zumindest einer Säure bzw. einer als Säure bzw. Protonendonator wirkenden Substanz nach einem der vorangehenden Ansprüche als Stabilisator für den Wirkstoff bzw. für eine flüssige pharmazeutische Zusammensetzung nach einem der vorangehenden Ansprüche, enthaltend den in einem nicht-wässrigen Lösungsmittel nach einem der vorangehenden Ansprüche gelösten Wirkstoff Alprostadil.

## Claims

1. A liquid pharmaceutical composition as a concentrate for producing an infusion solution, containing alprostadil as an active substance, dissolved in a non-aqueous solvent,
wherein the non-aqueous solvent is propylene glycol, 1,3 butanediol, ethyl acetate, ethanol 96% PhEur (95.1-96.9% (vol./vol.)) or water-free ethanol PhEur (min. 99.5% (vol./vol.)) or absolute ethanol or mixtures thereof,
and containing at least one acid and/or a substance acting as an acid and/or proton donor, as a stabiliser for the active substance,
the acid having a pKa1 value of < 4.85.

2. The composition according to claim 1, **characterised in that** the acid is an organic acid, in particular a carboxylic acid, and/or **in that** the acid is a hydroxycarboxylic acid.

3. The composition according to claim 1 or 2, **characterised in that** the acid is selected from the group consisting of malic acid, acetic acid, tartaric acid, lactic acid, acetylcysteine or citric acid, and/or **in that** the acid is a multivalent hydroxycarboxylic acid, preferably citric acid, malic acid or tartaric acid.

4. The composition according to any one of claims 1 to 3, **characterised in that** the acid is acetylcysteine, and/or **in that** the acid is malic acid, in particular DL-malic acid.

5. The composition according to claim 1, **characterised in that** the acid is an inorganic acid, in particular selected from the group comprising hydrochloric acid or phosphoric acid.

6. The composition according to any one of claims 1 to 5, **characterised in that** the acid has a pKa1 value in the range from 2 to 4.8.

7. The composition according to any one of claims 1 to 6, **characterised in that** the acid has a pKa1 value greater than or equal to 0 and less than 4.85 and is contained in a concentration of greater than or equal to c min = 1.45 * e^{(1.02* pKa1)} µM (= µmol/l) and less than or equal to c max = 11.75 * e^{(1.35*pKa1)} µM (= µmol/l),
or **in that** the acid has a pKa1 value of less than 0 and is contained in a concentration of greater than or equal to c min = 1.0 µM (= µmol/l) and less than or equal to c max = 15 µM (= µmol/l), preferably greater than or equal to c min = 1.3 µM (= µmol/l) and less than or equal to c max = 13.2 µM (= µmol/l).

8. The composition according to any one of claims 1 to 7, **characterised in that** the acid is contained in a concentration of 0.05-500 µg/ml, preferably 0.1-100 µg/ml, in particular 5-50 µg/ml.

9. The composition according to any one of claims 1 to 8, **characterised in that** the acid has a pKa1 value greater than or equal to 2 and less than or equal to 4.8 and is contained in a concentration of 0.5 to 500 µg/ml, in particular from 1 to 250 µg/ml.

10. The composition according to any one of claims 1 to 9, **characterised in that** the acid is acetic acid and is contained in a concentration between 20 and 500 µg/ml, in particular between 50 and 250 µg/ml,
or **in that** the acid is acetylcysteine and is contained in a concentration between 5 and 350 µg/ml, in particular between 10 and 100 µg/ml,
or **in that** the acid is malic acid, in particular DL malic acid, and is contained in a concentration between 5 and 200 µg/ml, in particular between 10 and 100 µg/ml,
or **in that** the acid is tartaric acid and is contained in a concentration between 2 and 150 µg/ml, in particular between 5 and 50 µg/ml.

11. The composition according to any one of claims 1 to 10, **characterised in that** the acid is phosphoric acid and is contained in a concentration between 0.5 and 15 µg/ml, in particular between 1 and 10 µg/ml,
or **in that** the acid is hydrochloric acid and is contained in a concentration between 0.1 and 0.8 µg/ml, in particular between 0.3 and 0.7 µg/ml.

12. The composition according to any one of claims 1 to 11, **characterised in that** alprostadil is contained in a concentration of 1-1000 µg/ml, preferably 5-750 µg/ml, in particular 20-500 µg/ml.

13. The composition according to any one of claims 1 to 12, **characterised in that** said composition is filled into glass ampoules, glass vials, cartridges or prefilled syringes.

14. Kit-of-parts, comprising the composition according to any one of claims 1 to 13, contained in a glass ampoule, glass vial, cartridge or prefilled syringe.

15. The composition according to any one of claims 1 to 13, **characterised in that** the pharmaceutical composition is a concentrate for producing a ready-to-use infusion solution for parenteral administration, in particular through a step of diluting with a suitable carrier solution, or is prepared as a concentrate for further manufacturing of a diluted, ready-to-use infusion solution for parenteral administration.

16. A ready-to-use parenteral composition, obtainable by diluting the concentrated composition according to one of the preceding claims with isotonic, aqueous solution, preferably with physiological saline solution, in particular in a ratio of 1:10 to 1:500, preferably 1:20 to 1:250.

17. A method for producing a liquid pharmaceutical composition according to one of the preceding claims, **characterised in that** to the active substance alprostadil, dissolved in a non-aqueous solvent according to one of the preceding claims, is added at least one acid and/or a substance acting as an acid and/or proton donor according to one of the preceding claims as a stabiliser for the active substance.

18. The use of at least one acid and/or a substance acting as an acid and/or proton donor according to one of the preceding claims as a stabiliser for the active substance and/or for a liquid pharmaceutical composition according to one of the preceding claims, containing the active substance alprostadil dissolved in a non-aqueous solvent according to one of the preceding claims.

## Revendications

1. Composition pharmaceutique liquide sous forme de concentré pour préparer une solution pour perfusion, contenant de l'alprostadil comme principe actif, dissous dans un solvant non aqueux,
le solvant non aqueux étant du propylène glycol, du 1,3-butanediol, de l'acétate d'éthyle, de l'éthanol à 96 % PhEur (95,1 à 96,9 % (v/v)) ou de l'éthanol anhydre PhEur (min. 99,5 % (v/v)) ou de l'éthanol absolu ou des mélanges de ceux-ci,
et au moins un acide ou une substance agissant comme un acide ou un donneur de protons en tant que stabilisant pour le principe actif,
l'acide présentant une valeur de pKs1 < 4,85.

2. Composition selon la revendication 1, **caractérisée en ce que** l'acide est un acide organique, en particulier un acide carboxylique et/ou **en ce que** l'acide est un hydroxyacide.

3. Composition selon l'une des revendications 1 à 2, **caractérisée en ce que** l'acide est choisi dans le groupe comprenant l'acide malique, l'acide acétique, l'acide tartrique, l'acide lactique, l'acétylcystéine ou l'acide citrique et/ou **en ce que** l'acide est un hydroxyacide polyvalent, de préférence l'acide citrique, l'acide malique ou l'acide tartrique.

4. Composition selon l'une des revendications 1 à 3, **caractérisée en ce que** l'acide est l'acétylcystéine et/ou **en ce que** l'acide est l'acide malique, en particulier l'acide DL-malique.

5. Composition selon la revendication 1, **caractérisée en ce que** l'acide est un acide inorganique, en particulier choisi dans le groupe comprenant l'acide chlorhydrique ou l'acide phosphorique.

6. Composition selon l'une des revendications 1 à 5, **caractérisée en ce que** l'acide présente une valeur de pKs1 dans la plage de 2 à 4,8.

7. Composition selon l'une des revendications 1 à 6, **caractérisée en ce que** l'acide présente une valeur de pKs1 supérieure ou égale à 0 et inférieure à 4,85 et est contenu à une concentration supérieure ou égale à c min = 1,45 * e ^{(1,02* pKs1)} µM (= µmol/l) et inférieure ou égale à c max = 11,75 * e^{(1,35*pKs1)} µM (= µmol/l),
ou **en ce que** l'acide présente une valeur de pKs1 inférieure à 0 et est contenu à une concentration supérieure ou égale à c min = 1,0 µM (= µmol/l) et inférieure ou égale à c max = 15 µM (= µmol/l), de préférence supérieure ou égale à c min = 1,3 µM (= µmol/l) et inférieure ou égale à c max = 13,2 µM (= µmol/l).

8. Composition selon l'une des revendications 1 à 7, **caractérisée en ce que** l'acide est contenu à une concentration de 0,05 à 500 µg/ml, de préférence de 0,1 à 100 µg/ml, en particulier de 5 à 50 µg/ml.

9. Composition selon l'une des revendications 1 à 8, **caractérisée en ce que** l'acide présente une valeur de pKs1 supérieure ou égale à 2 et inférieure ou égale à 4,8 et est contenu à une concentration de 0,5 à 500 µg/ml, en particulier de 1 à 250 µg/ml.

10. Composition selon l'une des revendications 1 à 9, **caractérisée en ce que** l'acide est l'acide acétique et est contenu à une concentration comprise entre 20 et 500 µg/ml, en particulier entre 50 et 250 µg/ml,
ou **en ce que** l'acide est l'acétylcystéine et est contenu à une concentration comprise entre 5 et 350 µg/ml, en particulier entre 10 et 100 µg/ml,
ou **en ce que** l'acide est l'acide malique, en particulier l'acide DL-malique et est contenu à une concentration comprise entre 5 et 200 µg/ml, en particulier entre 10 et 100 µg/ml,
ou **en ce que** l'acide est l'acide tartrique et est contenu à une concentration comprise entre 2 et 150 µg/ml, en particulier entre 5 et 50 µg/ml.

11. Composition selon l'une des revendications 1 à 10, **caractérisée en ce que** l'acide est l'acide phosphorique et est contenu à une concentration comprise entre 0,5 et 15 µg/ml, en particulier entre 1 et 10 µg/ml,
ou **en ce que** l'acide est l'acide chlorhydrique et est contenu à une concentration comprise entre 0,1 et 0,8 µg/ml, en particulier entre 0,3 et 0,7 µg/ml.

12. Composition selon l'une des revendications 1 à 11, **caractérisée en ce que** l'alprostadil est contenu à une concentration de 1 à 1000 µg/ml, de préférence de 5 à 750 µg/ml, en particulier de 20 à 500 µg/ml.

13. Composition selon l'une des revendications 1 à 12, **caractérisée en ce qu'**elle est contenue dans des ampoules en verre, des flacons en verre, des carpules ou des seringues préremplies.

14. Kit comprenant la composition selon l'une des revendications 1 à 13, contenue dans une ampoule en verre, un flacon en verre, une carpule ou une seringue préremplie.

15. Composition selon l'une des revendications 1 à 13, **caractérisée en ce que** la composition pharmaceutique est un concentré pour préparer une solution pour perfusion prête à l'emploi destinée à être administrée par voie parentérale, en particulier par le biais d'une étape de dilution avec une solution de support appropriée ou est adaptée sous forme de concentré pour la préparation supplémentaire d'une solution pour perfusion diluée prête à l'emploi destinée à être administrée par voie parentérale.

16. Composition prête à l'emploi à usage parentéral, pouvant être obtenue en diluant la composition concentrée selon l'une quelconque des revendications précédentes, avec une solution aqueuse isotonique, de préférence avec une solution saline physiologique, en particulier selon un rapport de 1/10 à 1/500, de préférence de 1/20 à 1/250.

17. Procédé pour préparer une composition pharmaceutique liquide selon l'une quelconque des revendications précédentes, **caractérisé en ce qu'**au moins un acide ou une substance agissant comme un acide ou un donneur de protons selon l'une quelconque des revendications précédentes, est ajouté(e), en tant que stabilisant pour le principe actif, au principe actif alprostadil dissous dans un solvant non aqueux selon l'une quelconque des revendications précédentes.

18. Utilisation d'au moins un acide ou une substance agissant comme un acide ou un donneur de protons selon l'une quelconque des revendications précédentes en tant que stabilisant pour le principe actif ou pour une composition pharmaceutique liquide selon l'une quelconque des revendications précédentes, contenant le principe actif alprostadil dissous dans un solvant non aqueux selon l'une quelconque des revendications précédentes.
